# EUROPÄISCHE PATENTSCHRIFT

(11) **EP 3 346 269 B1**
(45) Veröffentlichungstag und Bekanntmachung des Hinweises auf die Patenterteilung: **10.07.2019**
(21) Anmeldenummer: 17170586.6
(22) Anmeldetag: 11.05.2017
(51) Int. Cl.: G01N 33/18, G01N 31/02

(54) **TEST ZUR BESTIMMUNG DER SULFATKONZENTRATION**
TEST FOR MEASUREMENT OF SULFATE CONCENTRATION
ESSAI DESTINÉ À DÉTERMINER LA CONCENTRATION EN SULFATE

(30) Priorität: 10.01.2017 DE 102017200310
(43) Veröffentlichungstag der Anmeldung: 11.07.2018
(73) Patentinhaber: AXAGARIUS GmbH & Co. KG, 52355 Düren (DE)
(72) Erfinder: DICKSCHAT, Arne, 52355 Düren (DE); HOFFMANN, Dr. Jürgen, 52355 Düren (DE)
(74) Vertreter: dompatent von Kreisler Selting Werner - Partnerschaft von Patent- und Rechtsanwälten mbB

(56) Entgegenhaltungen:
- CN-A- 101 787 334
- US-A- 3 622 277
- US-A1- 2009 192 053
- US-A1- 2015 196 523
- US-A1- 2015 241 777

## Beschreibung

Die vorliegende Erfindung betrifft ein Verfahren zur Messung der Sulfatkonzentration einer Probe unter Verwendung einer Reagenzmischung umfassund ein wasserlösliches Bariumsalz, einen Ba²⁺-komplexierenden Chelator und ein kationisches und/oder nichtionisches Tensid. Ebenfalls offenbart ist weiterhin ein Testreagenzgefäß enthaltend diese Reagenzmischung und ein Testkit enthaltend die Reagenzmischung oder die Testreagenzgefäße.

Durch die Trinkwasserverordnung (TrinkwV 2001: siehe Anlage 3, Nr. 17 zu § 7 und § 14 Absatz 3) wird ein Grenzwert von 250 mg pro Liter vorgegeben. Sulfat ist ein essentieller Mineralstoff für den menschlichen Organismus. Zu hohe Sulfatgehalte im Trinkwasser wirken hingegen korrosiv. Daher muss der Sulfatgehalt im Trinkwasser kontinuierlich überprüft werden und ist daher ein fester Bestandteil der Klärprozeß- und Trinkwasseranalytik.

Methoden zur Bestimmung der Sulfatkonzentration mittels Ausfällung als schwerlösliches Bariumsulfat sind zahlreich bekannt und analytisch relevant. Das ausgefällte Bariumsulfat wird hierbei durch eine photometrische Messung quantitativ erfasst (z.B. durch ein turbidimetrisches Verfahren). Für eine Wasseranalytik sind diese Methoden als Schnelltest ausgestaltet, d.h. dass nach kontrollierter Vermischung der Probe mit dem Ba²⁺-haltigen Testreagenz durch manuelles Schütteln und einer zeitlich genau bemessenen Inkubation die auftretende Trübung im Photometer vermessen wird. Die auf dem Markt erhältlichen Schnelltests liefern allerdings recht ungenaue Ergebnisse, da das Ausmaß der Trübung von der Stärke und Dauer des manuellen Schütteins abhängt.

US 3,622,277 bezieht sich auf eine Methode zur Bestimmung der Sulfationenkonzentration von wässrigen Lösungen, Der Mechanismus der offenbarten Methode erfolgt über die Freisetzung ehemals Chelat-gebundener Metallionen unter sauren Bedingungen, die daraufhin mit den Sulfatanionen wechselwirken und als Niederschlag ausfallen.

Es besteht daher ein Bedarf an verbesserten Reagenzien und Testverfahren zur Bestimmung der Sulfationen-Konzentration.

Aufgabe der Erfindung ist es daher, ein gattungsgemäßes Testverfahren zur Bestimmung der Sulfatkonzentration dahingehend zu verbessern, dass es in seinen Messeigenschaften und insbesondere hinsichtlich der Methodenpräzision verbessert wird.

### Zusammenfassung der Erfindung

Diese Aufgabe wird gemäß der Erfindung gelöst durch ein
Testverfahren zur Bestimmung der Sulfatkonzentration in einer flüssigen Probe unter Bildung eines Präzipitats von Bariumsulfat mit einer Reagenzmischung als fester oder flüssiger Formulierung, wobei die Reagenzmischung umfasst:
I) mindestens ein wasserlösliches Bariumsalz;
II) mindestens einen Ba2+-komplexierenden Chelator;
III) mindestens ein kationisches und/oder nichtionisches Tensid,
   und das Testverfahren die folgenden Schritte umfasst:
   a) Bereitstellen eines Testreagenzgefäßes mit
      i. der flüssigen Formulierung als Testlösung; oder
      ii. mit der durch Solubilisierung der festen Formulierung erzielten gebrauchsfertigen Testlösung;
   b) Zugabe der Sulfationen-enthaltenden flüssigen Probe,
   c) Vermischen der Probe mit der Testlösung, und
   d) Vermessen der Probe auf Trübung in einem Nephelometer, Turbidimeter oder Photometer zur quantitativen Bestimmung des Sulfatkonzentration.

Bevorzugte Ausgestaltungen der Erfindung ergeben sich aus den abhängigen Ansprüchen.

Das erfindungsgemäße Testverfahren vereinigt mehrere entscheidende Vorteile gegenüber den aus dem Stand der Technik.

Wie die Erfinder festgestellt haben, führt die Anwesenheit eines Bariumionenkomplexierenden Chelators in Kombination mit einem kationischen und/oder anionischen Tensid zu einem Testverfahren mit stark erhöhter Methodenpräzision, insofern die Varianz der erhobenen Messwerte weitaus geringer ist. Diese geringe Varianz bleibt, im Gegensatz zu den aus dem Stand der Technik bekannten Meßmethoden, auch bei unterschiedlicher Schütteldauer erhalten. Das Reagenz erlaubt also nicht nur eine präzisere, sondern auch eine robustere Messung.

Weiterhin erlaubt das erfindungsgemäße Testverfahren die Vermessung in einem extrem breiten pH-Bereich, der von pH 2 bis pH 11 reicht.

Es hat sich herausgestellt, dass bereits mit sehr kurzer Schütteldauer von nur 5 bis 10 Sekunden eine hochpräzise Sulfatanalytik möglich ist.

Die daraus resultierende Messung stellt eine entscheidende Verbesserung in der WasserAnalytik dar, da sie auf einfache und unkomplizierte Weise eine präzisere Messung der Sulfationen-Konzentration möglich macht.

Die Reagenzmischung kann mit handelsüblichen Substanzen auf einfache Weise und zudem kostengünstig hergestellt werden.

Die Reagenzmischung kann ohne Mehraufwand in die bereits etablierten Testsysteme integriert werden.

Das erfindungsgemäße Testverfahren ist
einfach und unkompliziert und entspricht im Wesentlichen den etablierten Sulfat-Schnelltestverfahren.

### Ausführliche Beschreibung der Erfindung

Die im Rahmen des Testverfahren eingesetzte Reagenzmischung umfasst erfindungsgemäß drei Komponenten, nämlich 1.)
mindestens ein wasserlösliches Bariumsalz; 2.) mindestens einen Ba²⁺-komplexierenden Chelator und 3.) mindestens ein kationisches und/oder nichtionisches Tensid.

Die Barium-Kationen des wasserlöslichen Bariumsalz reagieren mit den Sulfat-Anionen unter Bildung von Bariumsulfat, das als schwerlösliches Salz ein Präzipitat bildet. Der Ba²⁺-komplexierenden Chelator komplexiert die Barium-Ionen und setzt sie erst durch Zugabe der sulfathaltigen Probe in kontrollierter Weise frei. In Verbindung mit dem Tensid entsteht sehr schnell eine gut quantifizierbare Trübung der Testlösung.

Nach einer Arbeitshypothese, auf die hier aber keine Festlegung erfolgen soll, wird durch die kontrollierte Freisetzung der einzelnen Barium-Kationen aus dem Chelatkomplex in Verbindung mit dem Tensid die Bildung von BaSO₄-Nanopartikeln mit homogener Größenverteilung induziert, die trotz schneller Präzipitatbildung eine gut quantifizierbare Trübung hervorrufen.

Erfindungsgemäß handelt es sich bei der zu vermessenden Probe um eine flüssige Probe, die bevorzugt eine wässrige Probe ist. Die Bestimmung der Sulfatkonzentration in wässrigem Milieu ist die bevorzugte Anwendungsform des erfindungsgemäßen Verfahrens, die gerade im Bereich der Trinkwasseranalytik von großer Bedeutung ist.

In bevorzugter Weise ist das mindestens eine wasserlösliche Bariumsalz ausgewählt aus der Gruppe enthaltend Bariumchlorid, Bariumphosphat, Bariumnitrat, Bariumacetat, Bariumbromid und Bariumhydroxid oder Kombinationen hiervon.

In einer besonders bevorzugten Ausführungsform der Erfindung wird als wasserlösliches Bariumsalz das Bariumchlorid verwendet, und dies zweckmäßigerweise in seiner Form als Dihydrat: BaCl₂ x 2H₂O. Das Bariumchlorid weist eine gute Wasserlöslichkeit auf und stellt ein günstiges Salz dar.

In einer Ausführungsform der Erfindung umfasst die Reagenzmischung genau ein Bariumsalz, das bevorzugt aus der oben aufgeführten Liste ausgewählt ist.

Erfindungsgemäß enthält die im Rahmen des Testverfahrens eingesetzte Reagenzmischung mindestens einen Ba²⁺ -komplexierenden
Chelator. Dem Fachmann sind zahlreiche Chelatoren bekannt, die er basierend auf ihrer Zähnigkeit und der Komplexbildungskonstante gegenüber Ba²⁺ für die erfindungsgemäße Reagenzmischung gezielt auswählen kann.

In einer bevorzugten Ausführungsform der Erfindung ist der mindestens eine Ba²⁺-komplexierende Chelator ausgewählt aus der Gruppe enthaltend Ethylendiamintetraessigsäure (EDTA), Diethylentriaminpentaessigsäure (DTPA), Ethylendiamindisuccinat (EDDS), Nitrilotriessigsäure, Ethylenglycol-bis(amino-ethylether)-tetraessigsäure (EGTA) oder Salze hiervon.

In besonders bevorzugter Weise wird als Ba²⁺-komplexierenden Chelator EDTA oder ein Salz hiervon verwendet. Dieser sechszähnige Ligand bildet stabile Komplexe mit Bariumkationen aus, ist günstig und von geringer Toxizität.

Als EDTA-Salze sind beispielsweise Dinatrium-ethylendiamin-tetraacetat (Na₂H₂EDTA), Tetranatrium-ethylendiamin-tetraacetat (Na₄EDTA) oder Calcium-dinatrium-ethylendiamin-tetraacetat (CaNa₂EDTA) einsetzbar.

Erfindungsgemäß umfasst die im Rahmen des Testverfahrens eingesetzte Reagenzmischung zudem mindestens ein kationisches
und/oder nichtionisches Tensid.

In einer Ausführungsform handelt es sich bei dem kationischen Tensid um ein quartäres Ammoniumsalz.

In einer bevorzugten Ausführungsform der Erfindung ist das kationische Tensid ausgewählt aus der Gruppe enthaltend Cetyltrimethylammoniumbromid (CTAB), Dodecyltriethylammoniumbromid (DTAB), Cetyltriethylammoniumbromid (CTEAP), Cetyltripropylammoniumbromid (CTPAB) und Tetrabutylammoniumbromid (TBAB), oder Kombinationen hiervon.

In einer besonders bevorzugten Ausführungsform wird als kationisches Tensid das CTAB verwendet. CTAB besitzt ausreichende Löslichkeit in Wasser und ist über mehrere Jahre in wässrigen Lösungen lagerstabil.

In einer Ausführungsform enthält die im Rahmen des Testverfahrens erfindungsgemäß eingesetzte Reagenzmischung das
kationische Tensid, das bevorzugt CTAB ist, in einem Anteil von zwischen 0,005 Gew. % und 0,5 Gew.-%, bevorzugt in einem Anteil von zwischen 0,01 Gew. % und 0,3 Gew.-%, und insbesondere bevorzugt in einem Anteil von 0,02 Gew. %.

In einer alternativen Ausführungsform handelt es sich bei dem kationischen Tensid um ein primäres, sekundäres oder tertiäres Amin oder ein lmin. Beispielhaft sei hier Octenidin genannt.

In einer Ausführungsform umfasst die Reagenzmischung ein oder mehrere nichtionische Tenside. Diese sind gemäß einer bevorzugten Ausführungsform ausgewählt aus der Gruppe enthaltend Fettalkoholethoxylate (FAEO) wie Brij35, Fettalkoholpropoxylate (FAPO), Alkylglucoside wie Tween 20, Alkylpolyglucoside (APG), Octylphenolethoxylate wie Octoxinol 9 oder Nonidet P40, oder Kombinationen hiervon.

In einer besonders bevorzugten Ausführungsform wird als nicht-ionisches Tensid das Octoxinol 9 verwendet. Octoxinol 9 ist ein p-tert-Octylphenol-Derivat mit einer Polyethylenglycol-Seitenkette aus 9 bis 10 Ethylenoxid-Einheiten. Es wird zum Beispiel unter den Markennamen Triton X-100 und Nonidet P40 vertrieben.

In einer Ausführungsform enthält die im Rahmen des Testverfahrens erfindungsgemäß eingesetzte Reagenzmischung das nichtionische Tensid, das bevorzugt Octoxinol-9 ist, in einem Anteil von zwischen 0,05 Gew. % und 10 Gew.-%, bevorzugt in einem Anteil von zwischen 0,1 Gew. % und 7,5 Gew.-%, und insbesondere bevorzugt in einem Anteil von zwischen 1 Gew. % und 5 Gew.-%.

In einer Ausführungsform der Erfindung liegen bei der Reagenzmischung die Bariumkationen des wasserlöslichen Bariumsalzes und der Ba²⁺-komplexierende Chelator in einem molekularen Verhältnis von zwischen 20:1 und 1:20, bevorzugt von zwischen 10:1 und 1:10, besonders bevorzugt von 5:1, 4:1, 3:1 oder 2:1 und insbesondere bevorzugt von 1,25:1 vor.

In einer bevorzugten Ausführungsform der Erfindung liegen somit das Bariumchlorid und das EDTA in einem molaren Verhältnis von 1,25:1 vor: EDTA : BaCl₂ x 2 H₂O = 1 : 1,25.

In einer Ausführungsform der Erfindung ist die Reagenzmischung dadurch gekennzeichnet, dass sie neben dem Bariumkationen keine signifikanten Mengen an Metallkationen enthält, die eine Chelatbildung mit dem Ba²⁺-komplexierenden Chelator eingehen, und bevorzugt keine signifikanten Mengen an Metallkationen enthalten, die ausgewählt sind aus der Gruppe enthaltend Mn(II), Cu(II), Fe(III), Pb(II), und Co(III), Al(III), Zn(II) und Cr(III). Unter einer signifikanten Menge ist hierbei eine Menge zu verstehen, bei der diese Metallkationen zu den Bariumkationen in einem molaren Verhältnis von weniger als 2:10, bevorzugt von weniger als 1:10 und besonders bevorzugt von weniger als 0,5 :10 stehen.

In einer weiteren Ausführungsform der Erfindung ist die Reagenzmischung dadurch gekennzeichnet, dass sie keine signifikante Mengen eines ein- oder mehrwertigen Alkohols enthält, und bevorzugt keine signifikante Mengen an Ethanol und Glycerin enthält. Unter einer signifikanten Menge ist hierbei eine Menge zu verstehen, bei der der einwertige oder mehrwertige Alkohol bezogen auf die Bariumkationen in einem molaren Verhältnis von weniger als 2:10, bevorzugt von weniger als 1:10 und besonders bevorzugt von weniger als 0,5 :10 steht.

Gemäß einer Ausführungsform handelt es sich bei der erfindungsgemäß im Rahmen des Testverfahrens eingesetzten Reagenzmischung um eine feste Formulierung.

In einer bevorzugten Ausführungsform ist die feste Formulierung ausgewählt ist aus der Gruppe enthaltend Granulat, Pulver, Tablette, Film, und Lyophilisat.

Besonders bevorzugt wird hier ein Lyophilisat eingesetzt. Obwohl dies aufwändiger in der Herstellung ist, haben die Erfinder hier gefunden, dass die Reagenzmischung in dieser Form nicht nur sehr lagerstabil ist, sondern sich auch schnell und reproduzierbar solubilisieren lässt, da der trockene poröse "Lyo-Kuchen" ein gutes Solubilisierungssubstrat darstellt. Da ein "Lyo-Kuchen" üblicherweise auf dem Gefäßboden adhäriert, ist auch gewährleistet, dass die Reagenzmischung in ihrer Gänze gelöst wird. Bei flüssigen Reagenzmischungen können Tropfen am Stopfen auftreten, die dann beim Öffnen des Gefäßes verspritzt werden können.

In einer Ausführungsform der Erfindung weist das Lyophilisat zusätzlich einen oder mehrere Gerüstbildner auf. Diese unterstützen die Ausbildung eines porösen Lyo-Kuchens.

In einer bevorzugten Ausführungsform ist der Gerüstbildner ein Saccharid und besonders bevorzugt ist der Gerüstbildner ausgewählt aus der Gruppe bestehend aus Mannitol, Saccharose, Trehalose, Dextran, Glucose und Stärke.

Insbesondere bevorzugt ist die Verwendung von Mannitol als Gerüstbildner.

Gemäß einer alternativen Ausführungsform handelt es sich bei der erfindungsgemäß im Rahmen der Erfindung eingesetzten Reagenzmischung um eine flüssige Formulierung. Dies hat den Vorteil, dass das
Reagenz für die Testung nicht erst solubilisiert werden muss, sondern direkt eingesetzt werden kann.

In einer bevorzugten Ausführungsform ist die flüssige Formulierung eine wässrige Lösung mit einem pH-Wert von zwischen 2 und 11, bevorzugt von zwischen 3 und 9 und besonders bevorzugt von zwischen 4 und 5. Die Erfinder haben hier herausgefunden, dass das Testverfahren unter Zuhilfenahme der Reagenzmischung in einem breiten pH-Bereich arbeitet, so beispielsweise in einem pH-Bereich von 2 bis 11.

Ebenfalls offenbart ist ein Testreagenzgefäß, das die erfindungsgemäß im Rahmen des Testverfahrens eingesetzte Reagenzmischung enthält.

In einer bevorzugten Ausführungsform ist das Testreagenzgefäß so ausgestaltet, dass es für eine Vermessung in einem Nephelometer, Turbidimeter oder Photometer geeignet ist.

In besonders bevorzugter Weise handelt es sich bei dem Testreagenzgefäß um ein Glasröhrchen mit kreisrundem Querschnitt.

Ebenfalls offenbart ist ein Testkit zur Bestimmung von Sulfat in Flüssigkeitsproben, wobei dieser Testkit eine erfindungsgemäß im

Rahmen des Testverfahrens eingesetzte Reagenzmischung oder ein oder mehrere Testreagenzgefäße enthält.

Hierbei ist die erfindungsgemäß im Rahmen des Testverfahrens eingesetzte Reagenzmischung in ihrer festen oder flüssigen
Formulierung bevorzugt in dem mindestens einen Testreagenzgefäß in einer für die Testung notwendigen Menge vorabgefüllt.

In einer alternativen Ausführungsform kann der Testkit das mindestens eine leere Testreagenzgefäß und die feste oder flüssige Formulierung lose in einem anderen Gefäß zur Verfügung stellen.

In einer bevorzugten Ausführungsform umfasst bei einer Reagenzmischung als fester Formulierung der Testkit zusätzlich ein Gefäß mit einer wässrigen Lösung zur Solubilisierung der festen Formulierung. Durch Zugabe der wässrigen Lösung, die bevorzugt destilliertes Wasser ist, kann die feste Reagenzmischung in eine gebrauchsfertige Testlösung überführt werden.

Die im Rahmen des Testverfahrens eingesetzte flüssige Probe ist bevorzugt eine Trinkwasserprobe oder eine Wasserprobe
aus einem Wasseraufbereitungsverfahren.

Zweckmäßigerweise sind vorhandene Trübungen der Sulfationen-enthaltenden flüssigen Probe vorab zu entfernen, beispielsweise durch Abfiltrieren.

Die flüssige Probe ist bevorzugt ausgewählt aus der Gruppe enthaltend Trinkwasser, Grundwasser, Oberflächenwasser, Mineralwasser, Abwasser, Nährlösungen zur Düngung, Boden nach entsprechender Probenvorbereitung, und Zugabewasser in der Bauindustrie.

In einer besonders bevorzugten Ausführungsform ist die flüssige Probe Trinkwasser, Oberflächenwasser oder Grundwasser.

In einer speziellen Ausführungsform ist die flüssige Probe kein Meerwasser.

### Definitionen

Unter einer "Reagenzmischung" wird im Rahmen der vorliegenden Erfindung eine Mischung verstanden, die die aufgeführten drei Substanzklassen umfasst. Die Mischung kann als Flüssigkeit oder als Feststoff vorliegen und erlaubt durch Inkontaktbringen mit einer sulfathaltigen flüssigen Probe die Ausbildung von schwerlöslichem Bariumsulfat.

Ein "Ba²⁺-komplexierender Chelator" im Sinne der Erfindung stellt einen chemische Verbindung dar, die als mehrzähniger Ligand mindestens zwei Koordinationsstellen (Bindungsstellen) des Ba²⁺-Zentralatoms einnimmt.

Unter "Tensiden" sind erfindungsgemäß alle Substanzen zu verstehen, die die Oberflächenspannung einer Flüssigkeit oder die Grenzflächenspannung zwischen zwei Phasen herabsetzen. Sie sind aus einem unpolaren und einem polaren Teil (funktionelle Gruppen) aufgebaut. Als unpolarer Teil dient meist eine Alkylgruppe oder eine Alkylbenzolgruppe.

Gemäß der Erfindung ist ein "kationisches Tensid" ein Tensid, das eine positiv geladene funktionelle Gruppe, jedoch nicht zusätzlich eine negativ geladene Gruppe besitzt. Wie jedes Tensid sind auch die kationischen Tenside aus einem polaren und einem unpolaren Teil aufgebaut. Als unpolarer Teil dienen verschiedene Alkylgruppen. Die polare Gruppe ist meistens eine quartäre Ammonium-Einheit, die bevorzugt ein Tetraalkylammoniumsalze bildet.

Unter einem "nichtionischen Tensid" ist ein Tensid zu verstehen, dass keine dissoziierbaren funktionellen Gruppen enthält und sich daher im Wasser nicht in Ionen auftrennt. Wie jedes Tensid sind auch die nichtionischen Tenside aus einem unpolaren und einem polaren Teil aufgebaut. Als unpolarer Teil dient meistens ein Fettalkohol (C12-C18) oder Octyl- oder Nonylphenole. Die polaren Gruppen sind üblicherweise Hydroxygruppen oder die (Poly)ethergruppen.

Unter "Selektivität" versteht man die Fähigkeit bestimmter Substanzen, aus einer Anzahl gebotener Möglichkeiten zur Reaktion eine bevorzugt auszusuchen. Die ausschließliche Auswahl bezeichnet man als Spezifität.

Als "Sensitivität" (Empfindlichkeit) bezeichnet man die Stärke der Änderung in der Antwort eines Messsignals geteilt durch die Änderung der auslösenden Größe (z.B. der Zielanalytkonzentration). Die Empfindlichkeit einer analytischen Methode entspricht der Steigung der Kalibrierkurve.

Im Rahmen der Erfindung sind die zu unterscheidenden Begriffe der "Systempräzision" (Messpräzision) und der "Methodenpräzision" wie folgt definiert: Die Messpräzision ist ein Maß für die Schwankungen, die durch die Testvorrichtung oder dem damit arbeitenden Analysengerät selbst verursacht werden. Sie wird durch die Mehrfachanalyse (z. B. sechsfach) eines Standards ermittelt. Die Forderung an die Messpräzision hängt vom Analysengerät ab. Dagegen beschreibt die Methodenpräzision die zufällige Streuung der Analysenergebnisse. Sie wird durch eine mehrfache (meist sechsfache) Durchführung der gesamten Analyse, d. h. vom Abwiegen über die Probenvorbereitung bis zu der Messung und Befund ermittelt (sechs Einwaagen realer Proben).

Die "Stabilität" der Testvorrichtung beinhaltet Lagerstabilität, Stabilität unter physikalischen Einflüssen wie z.B. Wärme, Licht, mechanische Beanspruchung.

Die "Richtigkeit" ist ein Maß für die Abweichung des Messwertes vom richtigen Wert (manchmal als "wahrer" Wert bezeichnet) aufgrund eines systematischen Fehlers. Die Richtigkeit wird im Allgemeinen durch den Vergleich mit einem Referenz- oder Arbeitsstandard (Soll/Ist-Vergleich), den Vergleich mit einer unabhängigen, möglichst validierten Methode oder durch das sogenannte Aufstocken ("Spiken" einer Probe) bestimmt. Wenn bei bestimmten Proben keine der drei Methoden anwendbar ist, kann als Kriterium für die Richtigkeit folgendes gelten: Die Selektivität ist erwiesen, Linearität ist vorhanden, und die die als Polynom berechnete Kalibrierkurve geht durch den Nullpunkt.

Die "Nachweisgrenze" ist die kleinste Konzentration (Menge) des Analyten in der Probe, die qualitativ noch erfasst werden kann (Ja/Nein-Entscheidung). Die "Bestimmungsgrenze" ist die kleinste Konzentration (Menge) des Analyten in der Probe, die mit gegebener Präzision und Richtigkeit quantitativ bestimmt werden kann. Das zugrunde liegende mathematische Modell und die Bestimmungsmethoden sind in der DIN 32645 beschrieben.

Die "Erfassungsgrenze" gibt die Konzentration (Menge) an, die mit einer Wahrscheinlichkeit von 50 % nachgewiesen werden kann. Somit kann die Erfassungsgrenze vereinfacht als die doppelte Nachweisgrenze angesehen werden.

### Ausführungsbeispiele

### 1. Flüssige Reagenzmischung mit kationischem Tensid

Es wird ein Reagenzmischung gemäß der folgenden Rezeptur hergestellt:

| Inhaltsstoffe | Menge |
|---|---|
| BaCl₂ * 2H₂0 | 1,83 g |
| EDTA | 2,19 g |
| CTAB | 200 mg |
| dest. Wasser | auf 100 mL auffüllen |

### 1.1. Testdurchführung

500 µL der gemäß Punkt 1 hergestellten Reagenzmischung werden in einer Rundküvette vorgelegt und anschließend 4,0 mL Probenlösung hinzugegeben. Der pH-Wert der Probe muss zwischen 2 und 11 liegen. Die Küvette wird verschlossen und die Lösungen werden durch manuelles Schütteln für 1 bis 30 Sekunden vermischt. Nach 15 minütiger Inkubation bei Raumtemperatur erfolgt eine Messung im Photometer bei 585 nm (für einen Messbereich von 20 bis 200 mg/L) oder bei 620 nm (für einen Messbereich von 40 bis 320 mg/L) oder bei 800 nm (für einen Messbereich von 60 bis 400 mg/L).

### 2. Flüssige Reagenzmischung mit nichtionischem Tensid

Es wird ein Reagenzmischung gemäß der folgenden Rezeptur hergestellt:

| Inhaltsstoffe | Menge |
|---|---|
| BaCl₂ * 2H₂0 | 1,83 g |
| EDTA | 2,19 g |
| Triton X 100 | 5 g |
| dest. Wasser | auf 100 mL auffüllen |

### 2.1. Testdurchführung

Die Testdurchführung geschieht gemäß dem in 1.1 beschriebenem Verfahren.

### 3. Gefriergetrocknete Reagenzmischung

Es wird ein Reagenzmischung gemäß der folgenden Rezeptur hergestellt:

| Inhaltsstoffe | Menge |
|---|---|
| BaCl₂ * 2H₂0 | 1,83 g |
| EDTA | 2,19 g |
| CTAB | 200 mg |
| Mannitol | 2,0 g |
| dest. Wasser | auf 100 mL auffüllen |

Von dieser Lösung werden je 1 mL in eine Rundküvette abgefüllt und einer Gefriertrocknung unterzogen.

### 3.1. Testdurchführung

Die Rundküvette mit der lyophilisierten Reagenzmischung wird geöffnet und mit 1 mL destilliertem Wasser versetzt, wieder verschlossen und durch kurzes Schütteln das Lyophilisat wieder resuspendiert. Anschließend werden 4,0 mL Probenlösung hinzugegeben. Der pH-Wert der Probe muss zwischen 2 und 11 liegen. Die Küvette wird verschlossen und die Lösungen werden durch manuelles Schütteln für 1 bis 30 Sekunden vermischt. Nach 15 minütiger Inkubation bei Raumtemperatur erfolgt eine Messung im Photometer bei 585 nm (für einen Messbereich von 20 bis 200 mg/L) oder bei 620 nm (für einen Messbereich von 40 bis 320 mg/L) oder bei 800 nm (für einen Messbereich von 60 bis 400 mg/L).

### 4. Vergleichsmessung zur Abhängigkeit von der Schütteldauer

In einer vergleichenden Messung wurden eine wässrige Lösung enthaltend 100 mg/L Sulfat mit einem herkömmlichen Barium-Schnelltest (s.u.) vermessen oder mit einem gefriergetrockneten erfindungsgemäßen Reagenz. Hierbei wurden die Proben unterschiedlich lange geschüttelt.

### 4.1 Herkömmlicher Barium-Schnelltest

Bei dem herkömmlichen Barium-Schnelltest wurde eine Rundküvette bereitgestellt, die 2 mL einer salzsauren Calciumchlorid-Natriumchlorid-Lösung enthält. Hierzu werden 4,0 mL der Probenlösung hinzugegeben, die Rundküvette verschlossen und durch Schütteln gemischt. Die Rundküvette wird in das Photometer eingesetzt und vermessen und der Messwert zu Null gesetzt. Danach wird ein gestrichener Messlöffel Bariumchlorid (entspricht ca. 200 mg Bariumchlorid x 2 H₂O) hinzugegeben, die Küvette nach der Zugabe sofort verschlossen und für 1, 5, 10 oder 30 Sekunden kräftig manuell geschüttelt. Nach zweiminütiger Inkubation wird die Rundküvette im Photometer bei 436 nm vermessen.

### 4.2 Barium-Schnelltest mit dem gefriergetrockneten Reagenz

Die Sulfatproben wurden anhand des 3.1 beschriebenen Verfahrens unter Verwendung der gefriergetrockneten Reagenzmischung gemäß Beispiel 3 behandelt. Hierbei wurde nach Zugabe der Probenlösung und Verschließen der Rundküvette die Mischung für 1, 5, 10 oder 30 Sekunden kräftig manuell geschüttelt. Zum Erhöhung der Messpräzision wurden vier unterschiedliche Photometer zur Messung verwendet. Für jede Schütteldauer wurden 8 Proben angesetzt, die dann paarweise in den vier unterschiedlichen Photometern zur Erhöhung der Messpräzision vermessen wurden.

### 4.2. Ergebnisse der photometrischen Messung

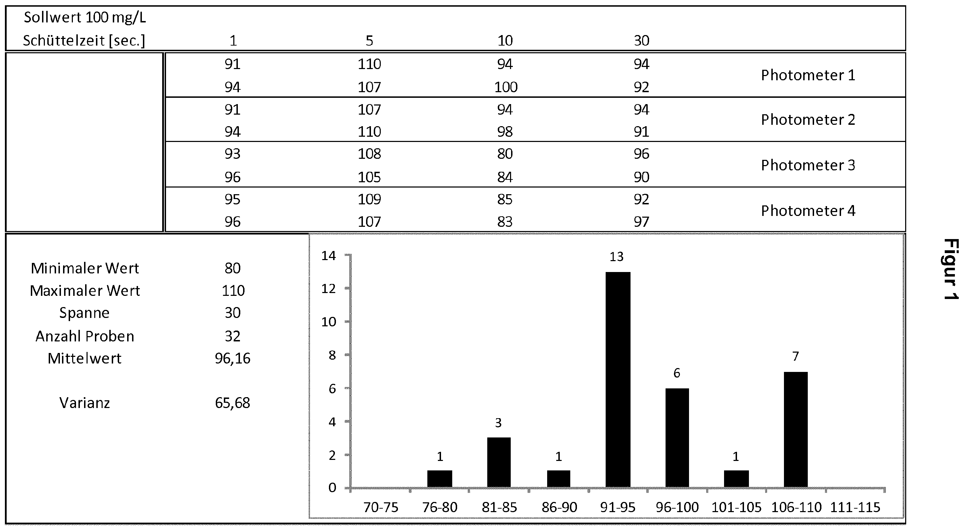

Die Ergebnisse der photometrischen Messung für den herkömmlichen Schnelltest sind in Figur 1 wiedergegeben. Die Messung ergab einen Mittelwert von 96,16 mg Sulfat/L, wich also um 3,84 mg/L vom Sollwert ab. Mit einem Maximalwert von 110 mg/L und einem Minimalwert von 80 mg/L wies der Messbereich eine Spanne von 30 mg/L auf. Die Grafik im unteren Bereich der Figur zeigt die Verteilung der Messwerte in diskreten Messwertbereichen von 5 mg/L.

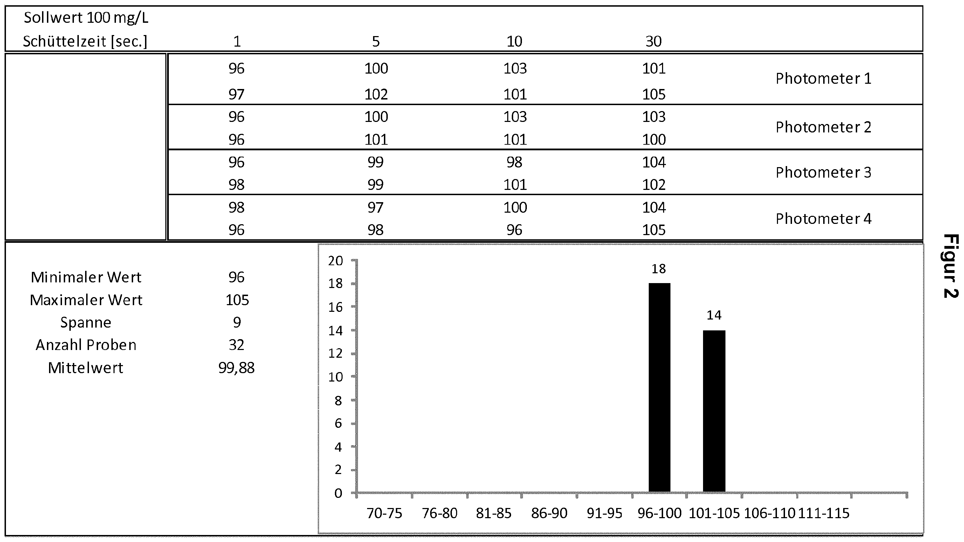

Die Ergebnisse der photometrischen Messung für den erfindungsgemäßen Schnelltest sind in Figur 2 wiedergegeben. Die Messung ergab einen Mittelwert von 99,88 mg Sulfat/L, wich also nur um 0,12 mg/L vom Sollwert ab. Mit einem Maximalwert von 105 mg/L und einem Minimalwert von 96 mg/L wies der Messbereich eine Spanne von 9 mg/L auf. Die Grafik im unteren Bereich der Figur zeigt die Verteilung der Messwerte in Messwertenbereichen von 5 mg/L.

Somit führt die erfindungsgemäße Reagenzmischung zu einem Messwert, der weitaus präziser ist und eine geringere Schwankungsbreite aufweist. Weiterhin sind die Messergebnisse weitgehend unabhängig von der Schütteldauer: Sie sind bei einer Schütteldauer von 5, 10 und 30 Sekunden sehr ähnlich. Es gibt hier beachtlicherweise keinen Trend zur Messwerterniedrigung, wie es bei dem herkömmlichen Verfahren der Fall ist (s. Figur 1, oben: 10- und 30-Sekundenmesswerte gegenüber 5-Sekundenmesswert).

### 5. pH-Abhängigkeit des erfindungsgemäßen Verfahrens

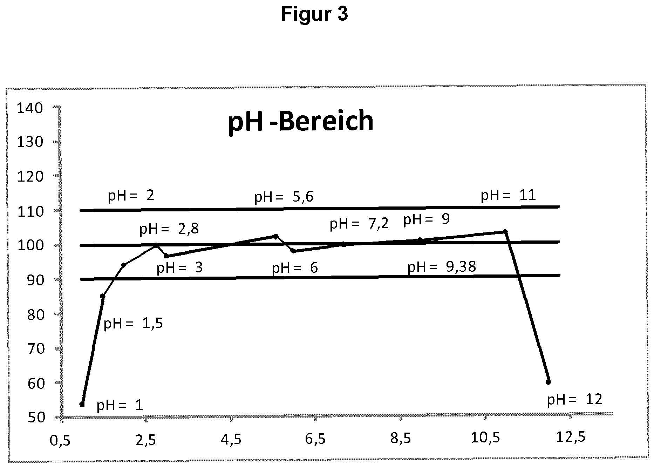

Es wurde eine erfindungsgemäße Reagenzmischung gemäß 3 hergestellt und Aliquote mit HCl, bzw. NaOH auf verschiedene pH-Werte eingestellt. Diese Reagenzmischungen wurden gemäß dem in 3.1 beschriebenen Verfahren zur Messung einer wässrigen Lösung enthaltend 100 mg/L Sulfat eingesetzt. Die Ergebnisse sind in Figur 3 dargestellt und zeigen, dass die Messung in einem pH-Wertbereich von 2 bis 11 sehr gute Ergebnisse liefert.

Ein möglicher Einsatz von zusätzlichen Metallkationen ist somit nicht erforderlich. Es wurde beschrieben, dass diese Metallkationen ein Ausfällen des EDTA in stark saurer Lösung unterbinden. Bei der erfindungsgemäßen Rezeptur wird eine Fällungsreaktion des EDTAs erst bei pH < 2 beobachtet.

### 6. Abhängigkeit des erfindungsgemäßen Verfahrens vom Detergens

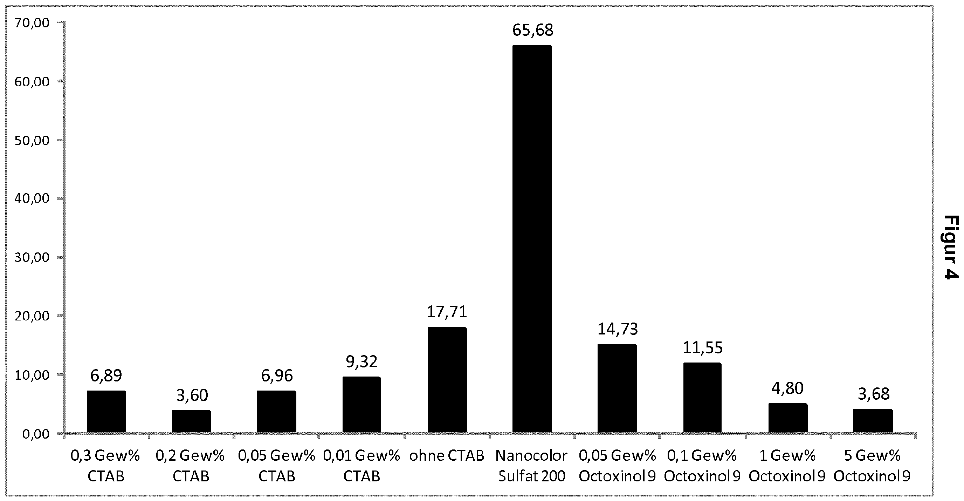

Es wurde eine erfindungsgemäße Reagenzmischung gemäß 1 und 2 hergestellt, wobei entweder das kationische Tensid CTAB oder das nicht-ionische Tensid Triton X-100 in verschiedenen Konzentrationen eingesetzt wurden. Diese Reagenzmischungen wurden gemäß dem in 1.1 und 2.1 beschriebenen Verfahren zur Messung einer wässrigen Lösung enthaltend 100 mg/L Sulfat eingesetzt. Die Ergebnisse sind in Figur 4 dargestellt und zeigen, dass alle Tensid-haltigen Mischungen gegenüber des Barium-Schnelltest aus dem Stand der Technik eine weitaus bessere Präzision aufweisen. Bei Triton x-100 erwies sich eine Konzentration von 5 Gew-% als optimal und bei CTAB eine Konzentration von 0,2 Gew.-%.

### Figurenlegenden

- Fig. 1: zeigt die Ergebnisse der Messung aus Beispiel 4 unter Verwendung des herkömmlichen Barium-Schnelltests. Im oberen Teil sind die Einzelergebnisse der Vermessung der 32 Proben dargestellt. Die Grafik im unteren Bereich ordnet die einzelnen Messwerte diskreten Messbereichen von je 5 mg/L zu.
- Fig. 2: zeigt die Ergebnisse der Messung aus Beispiel 4 unter Verwendung des erfindungsgemäßen Barium-Schnelltests. Im oberen Teil sind die Einzelergebnisse der Vermessung der 32 Proben dargestellt. Die Grafik im unteren Bereich ordnet die einzelnen Messwerte diskreten Messbereichen von je 5 mg/L zu.
- Fig. 3: zeigt die Ergebnisse der Messung aus Beispiel 5 unter Verwendung des erfindungsgemäßen Barium-Schnelltests mit unterschiedlichen pH-Werten.
- Fig. 4: zeigt die Ergebnisse der Messung aus Beispiel 6 unter Verwendung des erfindungsgemäßen Barium-Schnelltests. Hierbei wurden entweder das kationische Tensid CTAB oder das nicht-ionische Tensid Triton X-100 in jeweils unterschiedlichen Mengen eingesetzt.

Weitere Varianten der Erfindung und ihre Ausführung ergeben sich für den Fachmann aus der vorangegangenen Offenbarung, den Figuren und den Patentansprüchen.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

## Patentansprüche

1. Testverfahren zur Bestimmung der Sulfatkonzentration in einer flüssigen Probe unter Bildung eines Präzipitats von Bariumsulfat mit einer Reagenzmischung als fester oder flüssiger Formulierung, wobei die Reagenzmischung umfasst:
I) mindestens ein wasserlösliches Bariumsalz;
II) mindestens einen Ba²⁺-komplexierenden Chelator;
III) mindestens ein kationisches und/oder nichtionisches Tensid, und das Testverfahren die folgenden Schritte umfasst:
a) Bereitstellen eines Testreagenzgefäßes mit
i. der flüssigen Formulierung als Testlösung; oder
ii. mit der durch Solubilisierung der festen Formulierung erzielten gebrauchsfertigen Testlösung;
b) Zugabe der Sulfationen-enthaltenden flüssigen Probe,
c) Vermischen der Probe mit der Testlösung, und
d) Vermessen der Probe auf Trübung in einem Nephelometer, Turbidimeter oder Photometer zur quantitativen Bestimmung des Sulfatkonzentration.

2. Testverfahren nach Anspruch 1, **dadurch gekennzeichnet, dass** das Vermischen der Probe mit der Testlösung durch manuelles Schütteln für 1 bis 5 Sekunden erfolgt.

3. Testverfahren nach Anspruch 1 oder 2, **dadurch gekennzeichnet, dass** die flüssige Probe eine Trinkwasserprobe oder eine Wasserprobe aus einem Wasseraufbereitungsverfahren ist.

4. Testverfahren nach einem der vorangehenden Ansprüche, **dadurch gekennzeichnet, dass** das mindestens eine wasserlösliche Bariumsalz ausgewählt ist aus der Gruppe enthaltend Bariumchlorid, Bariumphosphat, Bariumnitrat, Bariumacetat, Bariumbromid und Bariumhydroxid oder Kombinationen hiervon.

5. Testverfahren nach einem der vorangehenden Ansprüche, **dadurch gekennzeichnet, dass** der mindestens eine Ba²⁺-komplexierende Chelator ausgewählt ist aus der Gruppe enthaltend Ethylendiamintetraessigsäure (EDTA), Diethylentriaminpentaessigsäure (DTPA), Ethylendiamindisuccinat (EDDS), Nitrilotriessigsäure, Ethylenglycol-bis(amino-ethylether)-tetraessigsäure (EGTA) oder Salze hiervon.

6. Testverfahren nach einem der vorangehenden Ansprüche, **dadurch gekennzeichnet, dass** das kationische Tensid ausgewählt ist aus der Gruppe enthaltend ein quartäres Ammoniumsalz wie Cetyltrimethylammoniumbromid (CTAB), Dodecyltriethylammoniumbromid (DTAB), Cetyltriethylammoniumbromid (CTEAP), Cetyltripropylammoniumbromid (CTPAB) und Tetrabutylammoniumbromid (TBAB).

7. Testverfahren gemäß einem der vorangehenden Ansprüche, **dadurch gekennzeichnet, dass** das nichtionische Tensid ausgewählt ist aus der Gruppe enthaltend Fettalkoholethoxylate (FAEO) wie Brij35, Fettalkoholpropoxylate (FAPO), Alkylglucoside wie Tween 20, Alkylpolyglucoside (APG), Octylphenolethoxylate wie Octoxinol 9.

8. Testverfahrengemäß einem der vorangehenden Ansprüche, **dadurch gekennzeichnet, dass** die Ba²⁺-Ionen des wasserlöslichen Bariumsalzes und der Ba²⁺-komplexierende Chelator in einem molekularen Verhältnis von zwischen 20:1 und 1:20, bevorzugt von zwischen 10:1 und 1:10 und besonders bevorzugt von 1,25:1 (BaCl₂*H₂O:EDTA) vorliegen.

9. Testverfahren gemäß einem der vorangehenden Ansprüche, **dadurch gekennzeichnet, dass** die Reagenzmischung neben dem Barium keine signifikanten Mengen an Metallkationen enthält, die eine Chelatbildung mit dem Ba²⁺-komplexierenden Chelator eingehen, und bevorzugt keine signifikanten Mengen an Metallkationen enthalten, die ausgewählt sind aus der Gruppe enthaltend Mn(II), Cu(II), Fe(III), Pb(II), und Co(III), Al(III), Zn(II) und Cr(III), wobei unter einer "signifikanten Menge" eine Menge zu verstehen ist, bei der diese Metallkationen zu den Bariumkationen in einem molaren Verhältnis von weniger als 2:10 stehen.

10. Testverfahren gemäß einem der vorangehenden Ansprüche, **dadurch gekennzeichnet, dass** die Reagenzmischung keine signifikanten Mengen eines ein- oder mehrwertigen Alkohols enthalten, und bevorzugt keine signifikanten Mengen an Ethanol und Glycerin enthalten, wobei unter einer "signifikanten Menge" eine Menge zu verstehen ist, bei der der ein- oder mehrwertige Alkohol bezogen auf die Bariumkationen in einem molaren Verhältnis von weniger als 2:10 steht.

11. Testverfahren gemäß einem der vorangehenden Ansprüche, **dadurch gekennzeichnet, dass** die feste Formulierung ausgewählt ist aus der Gruppe enthaltend Granulat, Pulver, Tablette, Film, und Lyophilisat.

12. Testverfahren gemäß Anspruch 11, **dadurch gekennzeichnet, dass** das Lyophilisat zusätzlich einen oder mehrere Gerüstbildner aufweist, der bevorzugt ein Saccharid ist und besonders bevorzugt ausgewählt ist aus der Gruppe bestehend aus Mannitol, Saccharose, Trehalose, Dextran, Glucose und Stärke.

13. Testverfahren gemäß einem der Ansprüche 1 bis 10, **dadurch gekennzeichnet, dass** die flüssige Formulierung eine wässrige Lösung mit einem pH-Wert zwischen 2 und 11, bevorzugt zwischen 3 und 9 und besonders bevorzugt zwischen 4 und 5 ist.

14. Testverfahren gemäß einem der vorangehenden Ansprüche, **dadurch gekennzeichnet, dass** die Reagenzmischung in einem Testreagenzgefäß enthalten ist.

15. Testverfahren gemäß Anspruch 14, **dadurch gekennzeichnet, dass** das Testreagenzgefäß für eine Vermessung in einem Nephelometer, Turbidimeter oder Photometer geeignet ist, und bevorzugt ein Glasröhrchen mit kreisrundem Querschnitt ist.

16. Testverfahren gemäß einem der vorangehenden Ansprüche, **dadurch gekennzeichnet, dass** es einen Testkit umfasst, wobei sich die feste oder flüssige Formulierung bereits im Testreagenzgefäß befindet, oder der Testkit ein leeres Testreagenzgefäß und die feste oder flüssige Formulierung lose in einem anderen Gefäß zur Verfügung stellt.

17. Testverfahren gemäß Anspruch 16, **dadurch gekennzeichnet, dass** es zusätzlich ein Gefäß mit einer wässrigen Lösung zur Solubilisierung der festen Formulierung umfasst, deren Zugabe bevorzugt eine gebrauchsfertige Testlösung ergibt.

## Claims

1. A test method for determining the sulfate concentration in a liquid sample with formation of a precipitate of barium sulfate using a mixture of reagents as a solid or liquid formulation, wherein said mixture of reagents includes:
I) at least one water-soluble barium salt;
II) at least one Ba²⁺-complexing chelating agent;
III) at least one cationic and/or non-ionic surfactant;
and the test method includes the following steps:
a) providing a test reagent vessel comprising
i. said liquid formulation as a test solution; or
ii. a ready-to-use test solution obtained by dissolving said solid formulation;
b) adding said liquid sample containing sulfate ions;
c) mixing the sample with the test solution; and
d) measuring the sample for turbidity on a nephelometer, turbidimeter or photometer for the quantitative determination of the sulfate concentration.

2. The test method according to claim 1, **characterized in that** said mixing the sample with the test solution is effected by manually shaking for 1 to 5 seconds.

3. The test method according to claim 1 or 2, **characterized in that** said liquid sample is a drinking water sample or a water sample from a water treatment method.

4. The test method according to any of the preceding claims, **characterized in that** said at least one water-soluble barium salt is selected from the group containing barium chloride, barium phosphate, barium nitrate, barium acetate, barium bromide, and barium hydroxide, or combinations thereof.

5. The test method according to any of the preceding claims, **characterized in that** said at least one Ba²⁺-complexing chelating agent is selected from the group containing ethylenediaminetetraacetic acid (EDTA), diethylenetriaminepentaacetic acid (DTPA), ethylenediamine disuccinate (EDDS), nitrilotriacetic acid, ethylene glycol-bis(aminoethylether)-tetraacetic acid (EGTA), or salts thereof.

6. The test method according to any of the preceding claims, **characterized in that** said cationic surfactant is selected from the group containing a quaternary ammonium salt, such as cetyltrimethylammonium bromide (CTAB), dodecyltriethylammonium bromide (DTAB), cetyltriethylammonium bromide (CTEAB), cetyltripropylammonium bromide (CTPAB), and tetrabutylammonium bromide (TBAB).

7. The test method according to any of the preceding claims, **characterized in that** said non-ionic surfactant is selected from the group containing fatty alcohol ethoxylates (FAEO), such as Brij 35, fatty alcohol propoxylates (FAPO), alkylglucosides, such as Tween 20, alkylpolyglucosides (APG), octylphenol ethoxylates, such as Octoxinol 9.

8. The test method according to any of the preceding claims, **characterized in that** the Ba²⁺ ions of said water-soluble barium salt and said Ba²⁺-complexing chelating agent are present at a molar ratio of from 20:1 to 1:20, preferably from 10:1 to 1:10, and more preferably 1.25:1 (BaCl₂·H₂O: EDTA).

9. The test method according to any of the preceding claims, **characterized in that** said mixture of reagents does not contain any significant amounts of metal cations that would undergo chelate formation with said Ba²⁺-complexing chelating agent except for barium, and preferably does not contain any significant amounts of metal cations selected from the group containing Mn(II), Cu(II), Fe(III), Pb(II), Co(III), AI(III), Zn(II), and Cr(III), wherein a "significant amount" means an amount in which these metal cations are at a molar ratio to the barium cations of less than 2:10.

10. The test method according to any of the preceding claims, **characterized in that** said mixture of reagents does not contain any significant amounts of a mono- or polyhydric alcohol, and preferably does not contain any significant amounts of ethanol and glycerol, wherein a "significant amount" means an amount in which said mono- or polyhydric alcohol is at a molar ratio of less than 2:10, based an the barium cations.

11. The test method according to any of the preceding claims, **characterized in that** said solid formulation is selected from the group containing granules, a powder, a tablet, a film, and a lyophilizate.

12. The test method according to claim 11, **characterized in that** said lyophilizate additionally includes one or more structuring agents, preferably a saccharide, more preferably selected from the group consisting of mannitol, sucrose, trehalose, dextran, glucose, and starch.

13. The test method according to any of claims 1 to 10, **characterized in that** said liquid formulation is an aqueous solution with a pH of from 2 to 11, preferably from 3 to 9, more preferably from 4 to 5.

14. The test method according to any of the preceding claims, **characterized in that** said mixture of reagents is contained in a test reagent vessel.

15. The test method according to claim 14, **characterized in that** said test reagent vessel is suitable for measurement in a nephelometer, turbidimeter or photometer, and preferably is a glass tube having a circular cross-section.

16. The test method according to any of the preceding claims, **characterized in that** it involves a test kit, wherein said solid or liquid formulation is already in the test reagent vessel, or the test kit provides an empty test reagent vessel and said solid or liquid formulation loosely in some other vessel.

17. The test method according to claim 16, **characterized in that** it additionally involves a vessel with an aqueous solution for dissolving the solid formulation, the addition of which preferably yields a ready-to-use test solution.

## Revendications

1. Procédure de test pour déterminer la concentration en sulfate dans un échantillon liquide par formation d'un précipité de sulfate de baryum, grâce à un mélange de réactifs en formulation solide ou liquide, dans lequel le mélange de réactifs comprend :
I) au moins un sel de baryum soluble dans l'eau ;
II) au moins un chélateur complexant Ba²⁺ ;
III) au moins un tensioactif cationique et/ou non ionique, et la procédure de test comprend les étapes suivantes consistant à :
a) préparer un récipient à réactif de test comportant
i. la formulation liquide en tant que solution de test ; ou
ii. la solution de test prête à l'emploi obtenue par solubilisation de la formulation solide ;
b) ajouter l'échantillon liquide contenant des ions sulfate,
c) mélanger l'échantillon avec la solution de test, et
d) mesurer la turbidité de l'échantillon dans un néphélomètre, un turbidimètre ou un photomètre pour déterminer de manière quantitative la concentration en sulfate.

2. Procédure de test selon la revendication 1, **caractérisée en ce que** le mélange de l'échantillon avec la solution de test a lieu par agitation manuelle pendant 1 à 5 secondes.

3. Procédure de test selon la revendication 1 ou 2, **caractérisée en ce que** l'échantillon liquide est un échantillon d'eau potable ou un échantillon d'eau issu d'un procédé de traitement de l'eau.

4. Procédure de test selon l'une des revendications précédentes, **caractérisée en ce que** le sel de baryum soluble dans l'eau, au moins au nombre de un, est choisi dans le groupe comprenant le chlorure de baryum, le phosphate de baryum, le nitrate de baryum, l'acétate de baryum, le bromure de baryum et l'hydroxyde de baryum, ou des combinaisons de ceux-ci.

5. Procédure de test selon l'une des revendications précédentes, **caractérisée en ce que** le chélateur complexant Ba²⁺, au moins au nombre de un, est choisi dans le groupe comprenant l'acide éthylènediaminetétraacétique (EDTA), l'acide diéthylènetriaminepentaacétique (DTPA), l'éthylènediamine disuccinate (EDDS), l'acide nitrilotriacétique, l'acide éthylèneglycol-bis(amino-éthyléther)-tétraacétique (EGTA) ou des sels de ceux-ci.

6. Procédure de test selon l'une des revendications précédentes, **caractérisée en ce que** le tensioactif cationique est choisi dans le groupe comprenant un sel d'ammonium quaternaire tel que le bromure de cétyltriméthylammonium (CTAB), le bromure de dodécyltriéthylammonium (DTAB), le bromure de cétyltriéthylammonium (CTEAP), le bromure de cétyltripropylammonium (CTPAB) et le bromure de tétrabutylammonium (TBAB).

7. Procédure de test selon l'une des revendications précédentes, **caractérisée en ce que** le tensioactif non ionique est choisi dans le groupe comprenant les éthoxylates d'alcool gras (FAEO), par exemple Brij35, les propoxylates d'alcool gras (FAPO), les alkylglucosides, par exemple Tween 20, les alkylpolyglucosides (APG), les éthoxylates d'octylphénol, par exemple Octoxinol 9.

8. Procédure de test selon l'une des revendications précédentes, **caractérisée en ce que** les ions Ba²⁺ du sel de baryum soluble dans l'eau et le chélateur complexant Ba²⁺ sont présents en un rapport moléculaire compris entre 20:1 et 1:20, de manière préférée compris entre 10:1 et 1:10, et de manière particulièrement préférée égal à 1,25:1 (BaCl₂*H₂O:EDTA).

9. Procédure de test selon l'une des revendications précédentes, **caractérisée en ce que**, hormis le baryum, le mélange de réactifs ne contient pas de quantités significatives de cations métalliques qui se chélatent avec le chélateur complexant Ba²⁺, et ne contient de manière préférée pas de quantités significatives de cations métalliques choisis dans le groupe comprenant Mn(II), Cu(II), Fe(III), Pb(II) et Co(III), Al(III), Zn(II) et Cr(III), où l'expression « quantité significative » doit être comprise comme désignant une quantité pour laquelle lesdits cations métalliques sont présents en un rapport molaire inférieur à 2:10 par rapport aux cations de baryum.

10. Procédure de test selon l'une des revendications précédentes, **caractérisée en ce que** le mélange de réactifs ne contient pas de quantités significatives d'un alcool univalent ou polyvalent, et ne contient de manière préférée pas de quantités significatives d'éthanol et de glycérol, où l'expression « quantité significative » doit être comprise comme désignant une quantité pour laquelle l'alcool univalent ou polyvalent est présent en un rapport molaire inférieur à 2:10 par rapport aux cations de baryum.

11. Procédure de test selon l'une des revendications précédentes, **caractérisée en ce que** la formulation solide est choisie dans le groupe comprenant granulé, poudre, comprimé, film et lyophilisât.

12. Procédure de test selon la revendication 11, **caractérisée en ce que** le lyophilisât présente en outre un ou plusieurs agent(s) structurant(s), qui est/sont de manière préférée un saccharide et est/sont de manière particulièrement préférée choisi(s) dans le groupe comprenant mannitol, saccharose, tréhalose, dextrane, glucose et amidon.

13. Procédure de test selon l'une des revendications 1 à 10, **caractérisée en ce que** la formulation liquide est une solution aqueuse ayant un pH compris entre 2 et 11, de manière préférée compris entre 3 et 9 et de manière particulièrement préférée compris entre 4 et 5.

14. Procédure de test selon l'une des revendications précédentes, **caractérisée en ce que** le mélange de réactifs est contenu dans un récipient à réactif de test.

15. Procédure de test selon la revendication 14, **caractérisée en ce que** le récipient à réactif de test est approprié pour une mesure dans un néphélomètre, un turbidimètre ou un photomètre, et est de manière préférée un tube en verre de section transversale circulaire.

16. Procédure de test selon l'une des revendications précédentes, **caractérisée en ce qu'**il comprend un kit de test, dans lequel la formulation solide ou liquide se trouve déjà dans le récipient à réactif de test, ou le kit de test fournit un récipient à réactif de test vide et la formulation solide ou liquide à part dans un autre récipient.

17. Procédure de test selon la revendication 16, **caractérisée en ce qu'**il comprend en outre un récipient comprenant une solution aqueuse destinée à la solubilisation de la formulation solide et dont l'ajout donne de manière préférée une solution de test prête à l'emploi.
